# EUROPÄISCHE PATENTSCHRIFT

(11) **EP 0 777 499 B1**
(45) Veröffentlichungstag und Bekanntmachung des Hinweises auf die Patenterteilung: **20.11.2002**
(21) Anmeldenummer: 95929003.2
(22) Anmeldetag: 18.08.1995
(51) Int. Cl.: A61K 48/00, A61P 35/00

(54) **LEBENDVAKZINE ZUR BEHANDLUNG VON TUMORERKRANKUNGEN**
LIVE VACCINE FOR THE TREATMENT OF TUMOUR DISEASES
VACCIN VIVANT UTILISE POUR TRAITER DES MALADIES TUMORALES

(30) Priorität: 24.08.1994 DE 4431401
(43) Veröffentlichungstag der Anmeldung: 11.06.1997
(73) Patentinhaber: MAX-DELBRÜCK-CENTRUM FÜR MOLEKULARE MEDIZIN, 13125 Berlin (DE)
(72) Erfinder: BLANKENSTEIN, Thomas, D-12203 Berlin (DE); CAYEUX-PEZZUTTO, Sophie, D-13469 Berlin (DE)
(74) Vertreter: Baumbach, Friedrich, Dr.
(86) Internationale Anmeldenummer: DE9501164
(87) Internationale Veröffentlichungsnummer: WO96005866

(56) Entgegenhaltungen:
- WO-A-93/21959
- NATURAL IMMUNITY, Bd. 13, Nr. 2-3, März 1994 - Mai 1994 BASEL, SCHWEIZ, Seiten 113-130, A. PORGADOR ET AL. 'Immunotherapy of tumor metastasis via gene therapy.'
- IMMUNOLOGY, Bd. 80, Nr. 3, November 1993 OXFORD, GROSSBRITANNIEN, Seiten 451-457, R. COSTELLO ET AL. 'Interleukin-7 is a potent co-stimulus of the adhesion pathway involving CD2 and CD28 molecules.'
- CELL, Bd. 71, 24.Dezember 1992 CAMBRIDGE, MA, VSA, Seiten 1093-1102, L. CHEN ET AL. 'Immunotherapy of tumor metastasis via gene therapy.'
- JOURNAL OF THE NATIONAL CANCER INSTITUTE, Bd. 85, Nr. 3, 3.Februar 1993 BETHESDA, MD, VSA, Seiten 207-216, A. BELLDEGRUN ET AL. 'Human renal carcinoma line transfected with interleukin-2 and/or interferon alpha gene(s): Implications for live cancer vaccines.'
- CURRENT OPINION IN IMMUNOLOGY, Bd. 4, Nr. 6, Dezember 1992 LONDON, GROSSBRITANNIEN, Seiten 619-623, D. PARDOLL 'New strategies for active immunotherapy with genetically engineered tumor cells.'
- EUROPEAN JOURNAL OF IMMUNOLOGY, Bd. 25, Nr. 8, August 1995 WEINHEIM, DEUTSCHLAND, Seiten 2325-2331, S. CAYEUX ET AL. 'Tumor cells cotransfected with interleukin-7 and B7.1 genes induce CD25 and CD28 on tumor-infiltrating T lymphocytes and are strong vaccines.'
- IMMUNOBIOLOGY, Bd. 191, Nr. 2-3, September 1994 STUTTGART, DEUTSCHLAND, Seiten 208-209, S. CAYEUX ET AL. 'Analysis of the vaccination-efficiency of cytokine/B7-transfected tumor cells.'

## Beschreibung

Die Erfindung betrifft eine Lebendvakzine gegen Tumorerkrankungen, ihre Herstellung und ihre Verwendung. Anwendungsgebiete sind die Medizin und die Gentechnik.

Vakzine gegen Tumorerkrankungen sind seit längerem bekannt. Die klassische und vielfach klinisch eingesetzte Vakzine besteht aus einem Gemisch von bestrahlten Tumorzellen und Adjuvantien wie beispielsweise Lysate von Bacillus Calmette-Guerin (BCG) oder Corynebacterium Parvum. Nach zwei Jahrzehnten klinischer Erprobung läßt sich zusammenfassen, daß diese Vakzine keine reproduzierbare Wirkung zeigt (siehe Übersichtsartikel: Oettgen, H. und Old, L., The History of Cancer Immunotherapy, *in*: Biological Therapy of Cancer, Eds. V. deVita, S. Hellman and S. Rosenberg, J. B. Lippincott Company 1991, S. 87-119). Jüngere Ergebnisse aus Tiermodellen haben gezeigt, daß der Transfer und die Expression mancher Zytokingene (z.B. IL2, IL4, IL7, TNF, IFNγ) das Wachstum der genmodifizierten Tumorzellen *in vivo,* nicht aber *in vitro* supprimieren kann. Diese Inhibition des Tumorwachstums ist das Ergebnis einer durch das transfizierte Zytokin induzierten Immunantwort. In vielen Fällen wird der gentransfizierte Tumor vollständig abgestoßen (siehe Übersichtsartikel: Blankenstein, Eur. J. Cancer, 1994, in press). In ähnlicher Weise kann die Expression des B7-Moleküls, ein Zelloberflächenprotein mit kostimulatorischer Aktivität, für T-Lymphozyten Tumorwachstums-inhibierend wirken. Von therapeutischer Bedeutung ist jedoch die Frage, ob die Abstoßung des gentransfizierten Tumors zu einem langzeitig anhaltenden immunologischen Gedächtnis für die Tumorzellen führt. Dies wäre dadurch zu erkennen, daß Tiere, die den gentransfizierten Tumor abgestoßen haben, später verabreichte nicht-gentransfizierte Tumorzellen ebenfalls abstoßen können. Dies ist auch in begrenztem Maße der Fall und, auf diesem Befund basierend, werden z.Z. mehrere klinische Studien durchgeführt, bei denen als Vakzine mit einem einzelnen Zytokingen versehene bestrahlte Tumorzellen eingesetzt werden (siehe Übersichtsartikel: Tepper, R. und Mule, J., 1994, Hum.

Gene Therapy 5, 153-164). Diese Studien werden als Gentherapiestudien bezeichnet.
In DE-OS 38 06 565 ist eine virusmodifizierte, tumorspezifische Vakzine beschrieben, die aus Tumorzellen vom Operationspräparat eines später zu behandelnden Patienten besteht, welche durch Bestrahlung inaktiviert und mit NDV-Virus unter sterilen Bedingungen inaktiviert wurden. Die Anwendung dieser Vakzine wurde gemäß DE-OS 39 22 444 verbessert, indem sie gemeinsam mit systemisch applizierten Zytokinen und ggf. mit Hämatopoese-stimulierenden Faktoren und/oder Anti-Suppressiva eingesetzt wird.

Der Nachteil der bisher bekannten Vakzine ist ihre geringe Wirksamkeit Diese Behauptung beruht zum einen auf früheren Befunden, die gezeigt haben, daß der Vakzineffekt einer mit einem einzelnen Zytokingen transfizierten Tumorzelle nicht besser ist als der, der sich durch ein Tumorzell/Adjuvant-Gemisch erzielen läßt (Hock et al., 1993, Cancer Res. 53, 714-716). Tumorzell/Adjuvant-Gemische sind wie oben gesagt als klinisch nicht wirksam nachgewiesen. Zum anderen führt weder die Expression eines einzelnen Zytokins noch die Expression des B7-Moleküls alleine zu einer zuverläßigen Tumorabstoßung. D.h. ein gewißer Prozentsatz der Mäuse, die gentransfizierte Zellen injiziert bekommen haben, entwickeln nach einer Latenzzeit einen Tumor, der häufig mit einem Verlust der Zytokinproduktion einhergeht (Hock et al., 1993, PNAS 90, 2774-2778). Dies verbietet es, Tumorzellen, die mit einem einzelnen immunstimulatorische Aktivität kodierenden Gen transfiziert wurden, als Lebendtumorzellvakzine zu verwenden.

Die Erfindung hat das Ziel, die Nachteile der bekannten Vakzine, die in ihrer unzureichenden Wirksamkeit in Verbindung mit der Notwendigkeit, proliferationsunfähige Tumorzellen zu spritzen, bestehen, zu beseitigen. Es soll gentechnisch eine Lebendvakzine entwickelt werden, die das Immunsystem gegen bereits im Körper vorhandene Tumorzellen stimuliert.

Dieses Ziel wird durch eine Vakzine gemäß Anspruch 1 realisiert, die Unteransprüche sind Vorzugsvarianten.

Ihre Herstellung erfolgt in autologen oder allogenen Systemen gemäß Anspruch 12 sowie ihre Verwendung gemäß der Ansprüche 13 bis 15.

Die erfindungsgemäße Lebendvakzine zur Behandlung von Tumorerkrankungen mit genmodifizierten Tumorzellen umfaßt ein Zytokin-Gen und ein immunstimulatorisches Membranprotein-Gen.
Es werden proliferationsfähige autologe oder allogene Tumorzellen eingesetzt, die zusätzlich ein oder mehrere Suizid-Gene enthalten können.

Unter Zytokinen werden Substanzen verstanden, die die Differenzierung, Proliferation und Aktivierung von Immunzellen induzieren. Gemäß der Erfindung kann die Lebendvakzine als Zytokin-Gen das Gen für Interleukin-2, Interleukin-4, Interleukin-7, Interferon oder Granulocyten-Makrophagen-Koloniestimulierenden Faktor(GM-CSF) umfassen; immunstimula-torische Membranprotein-Gene sind Proteine, die T-Zellen aktivieren, insbesondere das Gen des T-Zell-kostimulatorischen Moleküls B7.

Suizidgene sind Substanzen, die die Wirkstoffe in ein toxisches Produkt umwandeln, besonders bevorzugt ist das Thymidinkinasegen des Herpes-Simplex-Virus(HSV-TK)-Gen oder das Cytosindeaminase-gen.

Die Lebendvakzine findet Verwendung als Therapeutikum, insbesondere zur Behandlung von Tumorerkrankungen.

Ausgangsbasis für die Vakzine kann jede beliebige Tumorzelle (autolog oder allogen) sein. In diese Zelle werden drei therapeutische Gene eingebracht, bei diesem Gentransfer kann es sich um jede beliebige Methode (z.B. retroviralen Gentransfer) handeln. Jedes der drei therapeutischen Gene ist an einen konstitutiv wirkenden Promotor (z.B. Moloney Murine Leukemia Virus-Long Terminal Repeat, Elongation Factor 1, Cytomegalovirus) gekoppelt. Alle drei Gene integrieren stabil und zufallsmäßig ins Genom der Tumorzelle. Die drei therapeutischen Gene können wahlweise auf einem oder verteilt auf zwei Vektoren liegen. Der erfolgreiche Gentransfer wird durch zusätzliche auf den Vektoren befindliche positive Selektionsmarker (z.B. Neomycingen, Hygromycingen) nachgewiesen.
Das **erste** Gen ist ein Zytokingen (z.B. IL4, IL7). Die meisten Zytokine haben vielfältige Funktionen und induzieren Differenzierung, Proliferation und Aktivierung verschiedener Immunzellen. Die lokale Sekretion des transfizierten Zytokingens durch die Tumorzellen *in vivo* führt zu einer Entzündungsreaktion und einer Aktivierung der Immunzellen (u.a. T-Lymphozyten) gegen den Tumor. Das Ergebnis ist die Abstoßung des Tumors in den meisten, aber nicht in allen Fällen. Nur ein Teil der Tiere, die den Zytokingen-transfizierten Tumor abgestoßen haben, sind immun gegen den Tumor. Das **zweite** Gen kodiert für ein Zelloberflächenprotein mit immunstimulatorischer Aktivität (z.B. B7). B7 wird normalerweise auf antigenpräsentierenden Zellen ausgeprägt und dient über die Interaktion mit seinen Liganden CD28 oder CTLA-4 als kostimulatorisches Signal für die Aktivierung von T-Lymphozyten. In Abwesenheit von B7 werden über den T-Zellrezeptor stimulierte T-Lymphozyten in einen Zustand der Anergie getrieben. B7-Gentransfizierte Tumorzellen stimulieren *in vivo* eine T-Zell-vermittelte Immunantwort, die aber nur teilweise zur Tumorabstoßung führt und in einem moderaten Vakzineffekt resultiert. Das **dritte** Gen ist ein sogenanntes 'Suizid'-Gen (z.B. das Thymidinkinasegen des Herpes Simplex-Virus, HSV-TK). Die HSV-TK kann das nicht-toxische Gancyclovir in ein toxisches Produkt umwandeln. Dies erlaubt, durch systemische Gancyclovirgabe selektiv HSV-TK-exprimierende Tumorzellen abzutöten ohne normales Gewebe zu schädigen. Das HSV-TK-Gen dient als zusätzlicher Sicherheitsmarker zum Abschalten der Lebendtumorzellvakzine.

Die erfindungsgemäße gentechnisch hergestellte Tumorzellvakzine verliert ihre Wirksamkeit, wenn proliferationsunfähige Zellen verwendet werden (z.B. durch Bestrahlung oder Mitomycin C-Behandlung). Bei bisher an Menschen getestete Vakzinen wurden die Tumorzellen bestrahlt, da das Auswachsen der Vakzinzellen als Tumor ein Sicherheitsrisiko darstellte. Im Gegensatz zu den mit einem einzelnen Gen transfizierten Tumorzellen führt der Doppelgentransfer eines Zytokingens und des B7-Gens zu einer 100%igen Tumorabstoßung. Dieser durch die Erfindung erreichbare, überraschende synergistische Effekt zweier Gene, die immunstimulatorische Aktivität kodieren, ermöglicht den Einsatz einer Lebendzellvakzine, die durch die Option der Aktivierung des HSV-TK-Gens durch Gancyclovir zusätzlich gesichert ist. Die Vakzine zeichnet sich weiter dadurch aus, daß sie durch Zytokin- und B7-Gentransfer eine höhere Wirksamkeit bekommt im Vergleich zu jeweils nur mit einem der beiden Gene transfizierten Zellen.
Die Erfindung soll nachfolgend durch Ausführungsbeispiele näher erläutert werden.

### Anwendungsbeispiel

### 1. Expression von Zytokin-, B7- und HSV-TK-Genen in Tumorzellen

cDNAs für Zytokin-, B7- und HSV-TK-Gene können mit geeigneten Primern durch die Polymerasenkettenreaktion isoliert und in entsprechende {retrovirale) Vektoren kloniert werden. Mit Hilfe bekannter Verpackungszellinien (Pa317, Psi2) werden Retroviren hergestellt und mit diesen Maustumorzellen (Plasmacytom J558L und Mammaadenocarcinom TSA) infiziert. Der erfolgreiche Gentransfer wird durch Selektionsmarker (Neomycingen, Hygromycingen), die sich auf den Vektoren befinden, sichergestellt. Die Expression der Zytokingene wird durch kommerziell erhältliche ELISAs oder einen biologischen Assay nachgewiesen. IL4 kann beispielsweise durch die IL4-abhängige Proliferation von CT.4S-Zellen, IL7 durch die IL7-abhängige Proliferation der Zellinie IXN bestimmt werden. B7-Expression wird durch Färbung der Tumorzellen mit einem fluoreszensmarkierten anti-B7-Antikörper bestimmt. Die Expression des HSV-TK-Gens wird überprüft, indem man Gancyclovir (1-10 µg/ ml) für einen Zeitraum von 10-14 Tagen dem Kulturmedium beifügt und das Abtöten der Tumorzellen bestimmt. Es werden Zellinien hergestellt, die entweder nur IL4/ IL7 oder B7, bzw. beide Gene zusammen exprimieren. Zusätzlich enthalten die Zellen das HSV-TK-Gen.

### 2. Abstoßung der Tumorzellvakzine durch IL4/IL7 und B7

Vier Millionen J558L, J558-IL4, J558-B7, J558-IL4/B7, und 1 Millionen TSA, TSA-IL7, TSA-B7 und TSA-IL7/B7 Tumorzellen werden 6-8 Wochen alten syngenen BALB/c-Mäusen subkutan injiziert und das Tumorwachstum über einen Zeitraum von mindestens 6 Monaten verfolgt. Nicht-gentransfizierte oder Mock-transfizierte Tumorzellen wachsen in allen Fällen als Tumor. Zwischen 17.6 und 65% der Mäuse, die nur mit einem einzelnen Gen transfizierte Tumorzellen bekommen haben, entwickeln ebenfalls einen Tumor. In keinem der beiden Tumormodelle (J558L und TSA) waren IL4/IL7- und B7-kotransfizierte Tumorzellen auch nur in einem einzigen Fall in der Lage, als Tumor zu wachsen. Es wurden insgesamt 100 Mäuse analysiert. Die Ergebnisse sind in Tabelle 1 zusammengefaßt.

**Tabelle 1.**

| Abstoßung genmodifizierter Tumorzellen in BALB/c-Mäusen | | |
|---|---|---|
| Injizierte Tumorzellen | Anzahl der Mäuse mit Tumor/ Mäuse im Experiment | in% |
| J558L | 20/20 | 100 |
| J558-IL4 | 6/34 | 17.6 |
| J558-B7 | 14/59 | 23.7 |
| J558-IL4/B7 | 0/80 | 0 |
| | | |
| TSA | 20/20 | 100 |
| TSA-IL7 | 6/20 | 30 |
| TSA-B7 | 13/20 | 65 |
| TSA-IL7/B7 | 0/20 | 0 |

### 3. Abstoßung der Tumorzellvakzine durch Gancyclovir

Da die Zytokin/B7-gentransfizierten Tumorzellen zuverlässig abgestoßen wurden, wurde die HSV-TK-Genmarkierung als Sicherheitsmarker an nur mit demHSV-TK-Gen markierten TSA-Zellen getestet. Eine Million TSA- oder TSA-TK-Zellen wurden subkutan in BALB/c-Mäuse injiziert. Einen Tag später wurden die Mäuse für einen Zeitraum von 5 Tagen intraperitoneal mit 150 mg/ Kg Körpergewicht Gancyclovir oder Saline behandelt. Die Gancyclovir-Behandlung hatte keinen Einfluß auf das Tumorwachstum parentaler TSA-Zellen (10/10 Mäuse mit Tumor), TSA-TK-Zellen wuchsen als Tumor in nicht-behandelten Mäusen (10/10 Mäuse mit Tumor), wurden aber in Gancyclovir-behandelten Mäusen in den meisten Fällen eliminiert (2/10 Mäuse mit Tumor). Das HSV-TK-Gen wirkt somit bereits allein als Sicherheitsmarker und sollte gemeinsam mit dem oben beschriebenen synergistischen Zytokin/B7-Effekt ein zuverlässiges Abschalten der Lebendtumorzellvakzine sicherstellen.

### 4. Wirksamkeit der genmodifizierten Tumorzellvakzine

BALB/c-Mäuse wurden subkutan mit 4 Millionen Zellen immunisiert. Es gab Gruppen immunisiert mit J558L-, J558-IL4-, J558-B7-, J558-IL4/B7-Zellen und eine Gruppe mit J558L/C.Parvum-Adjuvant. Mit Ausnahme der J558L-Zellen, die durch Bestrahlung proliferationunfähig gemacht wurden, wurden alle Zellen lebend injiziert. Nach drei Wochen wurden den Mäusen kontralateral 4 Millionen parentale Tumorzellen ('Challenge'-Tumor) injiziert und das Tumorwachstum verfolgt. Das Ergebnis wie in Tabelle 2 dargestellt zeigt, daß der Vakzineffekt der J558-IL4/B7-Zellen größer war als der der J558-IL4- oder J558-B7-Zellen oder des Tumorzell/Adjuvant-Gemischs.

**Tabelle 2.**

| Vakzineffekt der genmodifizierten Tumorzellvakzine | | |
|---|---|---|
| Vakzinzellen | Mäuse mit 'Challenge'-Tumor/ Mäuse im Experiment | in % |
| keine | 20/20 | 100 |
| J558L, bestr. | 20/20 | 100 |
| J558-IL4 | 12/28 | 43 |
| J558-B7 | 18/48 | 38 |
| J558-IL4/B7 | 11/50 | 22 |
| J558/Adjuvant | | |

### 5. Vakzineffekt lebender gegenüber bestrahlten Tumorzellen

Alle bisher an Patienten eingesetzten Tumorzellvakzine wurden aus Sicherheitsgründen in bestrahlter Form verwendet, da mit einem einzelnen Gen transfizierte Tumorzellen oder Tumorzellen mit Adjuvant gemischt häufig als Tumor auswachsen. Der Befund, daß die oben beschriebenen dreifach-gentransfizierten Tumorzellen zuverlässig abgestoßen werden, ermöglicht, sie als Lebendvakzine einzusetzen. Dadurch wird die Wirksamkeit der Vakzine gesteigert. Immunisiert man nämlich BALB/c-Mäuse mit 4 Millionen lebenden, bzw. bestrahlten J558-IL4/B7-Zellen und injiziert 3 Wochen später 4 Millionen parentale J558-Zellen kontralateral, entwickeln 60% (6/10) der mit bestrahlten Zellen immunisierten Mäuse einen Tumor, aber 0% (0/10) der mit lebenden Zellen immunisierten Mäuse.

### 7. Vakzinierungseffektivität

Die Vakzinierungseffektivität IL-7/B7.1 koexprimierender Zellen ist höher als die nur mit einem einzelnen Gen transfizierenden Zellen und als die eines Tumorzell/Adjuvanz (C. parvum)-Gemisches.

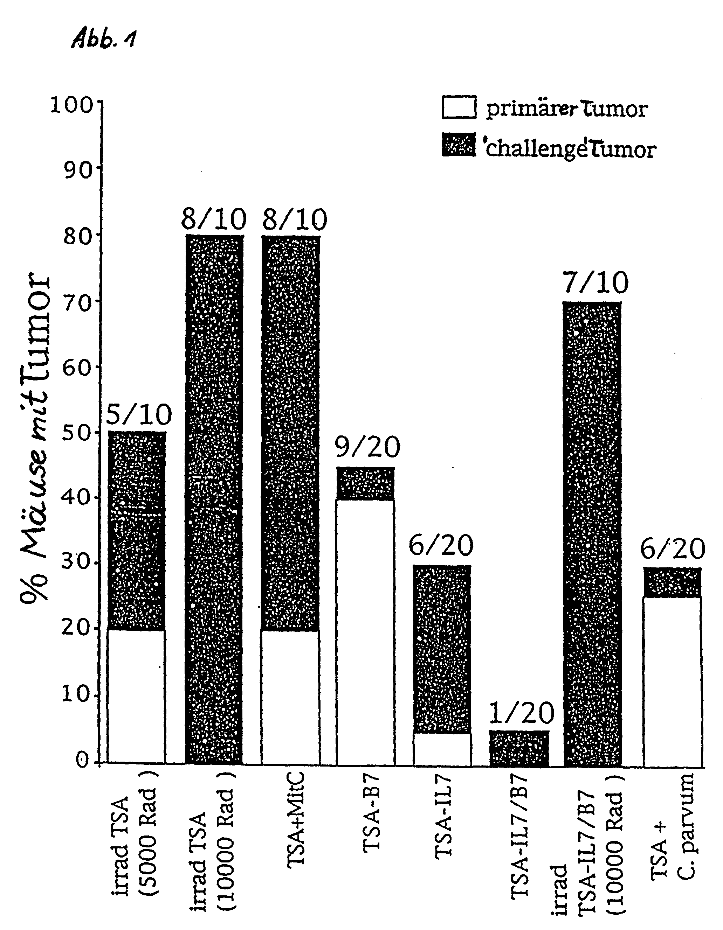

Um die Vakzinierungsstärke IL-7/B7.1 kotransfizierter TSA Zellen mit der des klinisch umfangreich getesteten Adjuvanz C. parvum oder mit nicht proliferierenden TSA-Zellen zu vergleichen, wurden Gruppen von Mäusen mit 2,5x10⁵ lebensfähigen TSA-IL7, -B7.1, -IL7/B7.1 oder Ursprungszellen gemischt mit C.parvum immunisiert. Zusätzlich wurden Mäuse mit bestrahlten (5000 oder 10000 Rad) TSA oder TSA-IL7/B7.1 Zellen oder TSA Zellen, die mit Mitomycin C (60µ/ml) behandelt wurden, immunisiert. Tumorfreien Mäusen wurden 2 Wochen später als Gegenprobe an einer anderen Stelle 2,5x10⁵ nichtmodifizierte Zellen injiziert ('Challenge'-Tumor). In Abb. 1 wird die Häufigkeit von Tumoren, und zwar der, die aus den Vakzinzellen und der, die aus den später verabreichten Parentalzellen herauswachsen, gezeigt. Das Tumorwachstum der Vakzinzellen wurde nur dann in allen. Mäusen verhindert, wenn die Zellen mit 10000 Rad bestrahlt wurden oder wenn IL-7/B7 kotransfizierte Zellen für die Immunisierung verwendet wurden. 80% (8/10) der Mäuse, die mit 10000 Rad bestrahlten Parentalzellen immunisiert wurden, und 30% (3/10) der Mäuse, die mit 5000 Rad bestrahlten Parentalzellen immunisiert wurden, entwickelten einen Tumor von den später verabreichten Parentalzellen. In der letztgenannten Gruppe entwickelten 20% (2/10) einen Tumor von den Vakzinzellen. In analoger Weise entwickelten 80% (8/10) der Mäuse, die mit Mitomycin C behandelten TSA-Zellen immunisiert waren, einen Tumor (20% primären Tumor, 60% 'challenge' Tumor). In der Tumorzell/C.parvum-Gruppe entwickelten 25% (5/20) der Mäuse einen Tumor von den Vakzinzellen und 5% (1/20) von den 'Challenge'-Zellen. Von denjenigen Mäusen, die die TSA-B7.1 Vakzinzellen abgestoßen hatten (60%, 12/20), entwickelten 5% (1/20) einen 'challenge' - Tumor. Im Gegensatz dazu entwickelten die mit TSA-IL7 vorbehandelten Mäuse in 25% der Fälle (5/20) einen 'Challenge'-Tumor und 5% (1/20) einen Tumor von den Vakzinzellen. Mit anderen Worten führte B7 exprimiert durch die Tumorzellen zu einer vergleichsweise schlechten Tumorabstoßung, aber gutem Vakzineffekt, während IL-7 bessere Abstoßung der Vakzinzellen bewirkte, aber einen schlechteren Vakzineffekt. B7.1 und IL-7 aktivieren das Immunsytsem daher in verschiedener und komplementärer Weise. Da nur TSA-IL-7/B7.1 Vakzinzellen in allen Mäusen vollständig abgestoßen wurden und einen Schutz gegenüber Tumorwachstum der später verabreichten Parentalzellen in 19/20 (95%) der Mäuse verursachten, wirken IL-7 und B7 in synergistischer Weise.
Sämtliche der oben genannten Immunisierungsexperimente mit transfizierten Tumorzellen wurden mit lebenden Zellen durchgeführt. Es wurde weiterhin der Vakzinierungseffekt von lebenden TSA-IL-7/B7 Zellen, die für die Immunisierung verwendet wurden, mit dem der selben Zellen verglichen, die vor der Injektion mit 10000 Rad bestrahlt wurden. 95% (19/20) der Mäuse, die mit lebenden Zellen, aber nur 30% (3/10) der Mäuse, die mit bestrahlten Zellen immunisiert waren, waren in der Lage, den 'Challenge'-Tumor abzustoßen (Abb. 1). Die beschriebene Vakzine erhält ihre Wirksamkeit daher durch den synergistischen Effekt von IL-7 und B7 und die Verwendung von proliferationsfähigen Zellen.

### 8. Phänotypische Beschreibung von T-Lymphocyten in transfizierten Tumoren und das Wachstum von Tumorzellinien in nackten und SCID-Mäusen

Zur Untersuchung des zellulären Mechanismus der IL-7/B7.1 induzierten Tumorabstoßung wurde eine Immunofluoreszenz-Analyse der den Tumor infiltrierenden T-Zellen durchgeführt. Dazu wurden parentale TSA-Zellen, TSA-IL7-, TSA-B7.1- und TSA-IL7/B7.1-Zellen subkutan in Balb c Mäuse injiziert und nach 6, 8, und 10 Tagen die Tumorknötchen isoliert, Einzelzellsuspensionen präpariert und Zellen gefärbt unter Verwendung der Immunofluoreszenz mit mAbs gegen CD4, CD8, CD25 und CD28. Die Prozentraten von CD4⁺ und CD8⁺ Zellen unter den infiltrierten Zellen sind in Tabelle 4 gezeigt, während die Prozentraten von CD4⁺ und CD8⁺ Zellen, die mit CD28⁺ und CD25⁺ (p55 IL2 Rezeptor)koexprimiert werden, in Tabelle 5 gezeigt sind. Sowohl CD4⁺ und CD8⁺ T Zellen waren in TSA-B7 vermehrt in Vergleich zu den Parentaltumoren. In TSA-IL7 Tumoren war ein Ansteigen von CD4⁺ T Zellen zu beobachten. T Zellen (CD4⁺ und CD8⁺) waren nicht weiter erhöht in IL7/B7 transfizierten Tumoren. Jedoch offenbart die Doppelfluoreszenz-Färbung für die T Zell-Subtypenmarker CD4 und CD8 sowie die Aktivierungsmarker CD28 und CD25 phänotypisch verschiedene T Zellen in IL7 oder B7 transfizierten Tumoren. In TSA-B7 Tumoren sind die T-Zellen (CD4⁺ und CD8⁺) zu einem hohen Prozentsatz CD28⁺, aber die meisten T Zellen sind CD25⁻. Im Gegensatz dazu sind die T-Zellen in TSA-IL7 Tumoren hauptsächlich CD25⁺ und CD28⁻ Zellen im wesentlichen abwesend. Zum Vergleich wurden in Parentaltumoren nur wenige CD28⁺ und so gut wie keine CD25⁺ T Zellen nachgewiesen. Wichtig ist, daß in TSA-IL7/B7 Tumoren die meisten CD4⁺ und CD8⁺ Zellen CD25⁺ und CD28⁺ sind. In Zusammenhang mit der Tatsache, daß nur IL7/B7 kotransfizierte Zellen zuverlässig abgestoßen wurden und eine sehr starke systemische Tumorimmunität induzierten (siehe oben), ist die lokale IL-7 Sekretion und B7 Expression durch die Tumorzellen in besonderem Maße geeignet, die Aktivierung der den Tumor infiltrierenden Lymphozyten zu bewirken. Zur Demonstration, daß die konzertierte Tumorsuppressionsaktivität von IL7 und B7 einzig und allein über T Zellen eintritt, wurden IL-7, B7, IL-7/B7 transfizierte TSA Zellen und zum Vergleich parentale oder mit einem Kontrollvektor transfizierte TSA Zellen in nackte und SCID-Mäuse injiziert und die Tumorwachstumskinetiken wurden verglichen. Wie in Tabelle 6 zu sehen ist, ist weder die IL-7 Sekretion noch die B7 Expression durch Tumorzellen noch beide zusammen in der Lage, die Tumorbildung in einem der immundefizienten Mäusestämme zu verzögern, was die absolute Erfordernis von T-Zellen für die IL-7 und B7 induzierte Antitumor Immunantwort belegt.

**Tabelle 3**

| Prozentraten von CD4⁺ und CD8⁺ Zellen unter den tumorinfiltrierenden Zellen | | |
|---|---|---|
| Tumorzellinie | % positive Zellen* | |
| | CD4⁺ | CD8⁺ |
| TSA | 15.7⁺/-4.0 | 9.7⁺/-5.0 |
| TSA-B7.1 | 46.3⁺/-2.1 | 24.0⁺/-3.6 |
| TSA-IL7 | 30.0⁺/-7.2 | 10.0⁺/-1.7 |
| TSA-IL7/B7.1 | 28.6⁺/-4.0 | 14.3⁺/-9.3 |

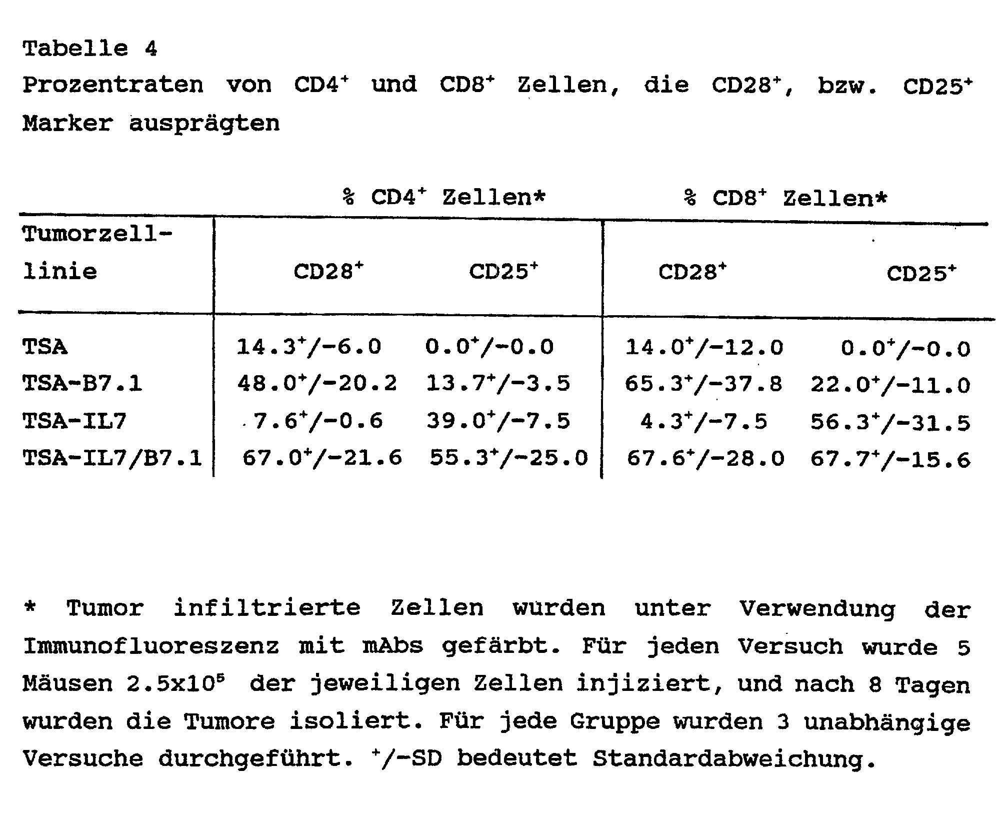

**Tabelle 5**

| Analyse des Tumorwachstums von TSA Tumorzellinien in immunodefizienten Mäusen | | |
|---|---|---|
| Tumorzellinien | nu/nu | SCID |
| TSA | 5/5 (19⁺/-2) | 5/5 (20⁺/-2) |
| TSA-TK | 5/5 (19⁺/-1) | 5/5 (20⁺/-1) |
| TSA-B7.1 | 5/5 (21⁺/-2) | 5/5 (20⁺/-2) |
| TSA-IL7 | 5/5 (22⁺/-3) | 5/5 (22⁺/-1) |
| TSA-IL7/B7.1 | 5/5 (22⁺/-0) | 5/5 (21⁺/-1) |

Die genannten Zellen (2.5x10⁵) wurden subkutan in die Mäusestämme injiziert. Tumorinzidenz und Tumorlatenz (in Klammern) sind gegeben.

## Patentansprüche

1. Lebendvakzine zur Behandlung von Tumorerkrankungen mit genmodifizierten Tumorzellen umfassend
- ein Zytokin-Gen und
- ein immunstimulatorisches Membranprotein-Gen.

2. Lebendvakzine nach Anspruch 1, wobei die Tumorzellen zusätzlich ein oder mehrere Suizid-Gene enthalten.

3. Lebendvakzine nach Anspruch 1 und 2, wobei die Tumorzellen
- ein Zytokin-Gen,
- ein immunstimulatorisches Membranprotein-Gen und
- ein Suizid-Gen
enthalten.

4. Lebendvakzine nach einem der Ansprüche 1-3, wobei als Tumorzellen proliferationsfähige autologe oder allogene Tumorzellen eingesetzt werden.

5. Lebendvakzine nach Anspruch 1-4, wobei das Zytokin-Gen durch Transfektion erhalten wurde.

6. Lebendvakzine nach Anspruch 1-5, wobei Zytokine Substanzen sind, die die Differenzierung, Proliferation und Aktivierung von Immunzellen induzieren.

7. Lebendvakzine nach Anspruch 1-6 , wobei die Tumorzellen als Zytokin-Gen umfassen
das Gen für Interleukin-2, Interleukin-4, Interleukin-7, Interferon- oder Granulocyten-Makrophagen-Kolonie-stimulierenden Faktor (GM-CSF).

8. Lebendvakzine nach Anspruch 1-7, wobei die immunstimulatorischen Membranprotein-Gene Proteine codieren, die T-Zellen aktivieren.

9. Lebendvakzine nach Anspruch 1,3 und 8, wobei das immunstimulatorische Membranprotein-Gen das Gen des T-Zell-kostimulatorischen Moleküls B7 ist.

10. Lebendvakzine nach Anspruch 1 bis 3, wobei Suizidgene Substanzen sind, die Wirkstoffe in ein toxisches Produkt umwandeln.

11. Lebendvakzine nach Anspruch 1 bis 3 oder 10, wobei das Suizidgen das Thymidinkinasegen des Herpes-Simplex-Virus(HSV-TK)-Gen oder das Cytosindeaminasegen ist.

12. Verfahren zur Herstellung einer Lebendvakzine nach einem der Ansprüche 1 bis 11 in autologen oder allogenen Systemen.

13. Lebenvakzine nach einem der Ansprüche 1 bis 11 zur Verwendung als Therapeutikum.

14. Verfahren zur Herstellung von Lebendvakzinen nach einem der Ansprüche 1 bis 11 zu Behandlung von Tumorerkrankungen.

## Claims

1. Live vaccines for treatment of tumour diseases with gene-modified tumour cells entailing
- a cytokine gene and
- an immune-stimulatory membrane protein gene.

2. Live vaccines according to Claim 1 wherein the tumour cells additionally contain one or more suicide genes.

3. Live vaccines according to Claims 1 and 2, wherein the tumour cells contain
- a cytokine gene,
- an immune-stimulatory membrane protein gene and
- a suicide gene.

4. Live vaccines according to one of the Claims 1-3, wherein proliferation-capable autologous or allogenic tumour cells are used as the tumour cells.

5. Live vaccines according to Claims 1-4, wherein the cytokine gene has been obtained by transfection.

6. Live vaccines according to Claims 1-5, wherein cytokines are substances inducing the differentiation, proliferation and activation of immune cells.

7. Live vaccines according to Claims 1-6, wherein the tumour cells entail as a cytokine gene
the gene for Interleukin-2, Interleukin-4, Interleukin-7, Interferon or granulocyte/macrophage colony-stimulating factor (GM-CSF).

8. Live vaccines according to Claims 1-7, wherein the immune-stimulatory membrane protein genes code proteins activating T-cells.

9. Live vaccines according to Claims 1, 3 and 8, wherein the immune-stimulatory membrane protein gene is the gene of the T-cell co-stimulatory molecule B7.

10. Live vaccines according to Claims 1-3, wherein suicide genes are substances converting the active agents into a toxic product.

11. Live vaccines according to Claims 1 to 3 or 10, wherein the suicide gene is the thymidinikase gene or the herpes simplex virus (HSV-TK) gene or the cytosindeaminase gene.

12. Method for the production of a live vaccine according to one of the claims 1 to 11 in autologous or allogenic systems.

13. Live vaccines according to one of the claims 1 to 11 for use as a therapeutic.

14. Method for the production of live vaccines according to one of Claims 1 to 11 for treatment of tumour diseases.

## Revendications

1. Vaccins vivants pour le traitement de maladies tumorales présentant des cellules tumorales modifiées génétiquement englobant
- un gène de cytokine et
- un gène de la protéine de membrane stimulant la réponse immunitaire.

2. Vaccins vivants selon la revendication 1, se caractérisant par le fait que les cellules tumorales contiennent en plus un ou plusieurs gènes suicides.

3. Vaccins vivants selon la revendication 1 et 2, se caractérisant par le fait que les cellules tumorales contiennent
- un gène de cytokine,
- un gène de la protéine de membrane stimulant la réponse immunitaire et
- un gène suicide.

4. Vaccins vivants selon l'une des revendications 1-3, se caractérisant par le fait que des cellules tumorales autologues ou allogènes capables de proliférer sont utilisées comme cellules tumorales.

5. Vaccins vivants selon la revendication 1-4, se caractérisant par le fait que le gène de cytokine a été obtenu par transfection.

6. Vaccins vivants selon la revendication 1-5, se caractérisant par le fait que les cytokines sont des substances qui induisent la différentiation, la prolifération et l'activation de cellules immunitaires.

7. Vaccins vivants selon la revendication 1-6, se caractérisant par le fait que les cellules tumorales englobent en tant que gène de cytokine
le gène pour l'interleukine-2, l'interleukine-4, l'interleukine-7, le facteur de croissance de l'interféron ou de croissance des colonies de granulocytes-macrophages (GM-CSF).

8. Vaccins vivants selon la revendication 1-7, se caractérisant par le fait que les gènes de la protéine de membrane stimulant la réponse immunitaire codent des protéines qui activent les cellules T.

9. Vaccins vivants selon la revendication 1, 3 et 8, se caractérisant par le fait que le gène de la protéine de membrane stimulant la réponse immunitaire est le gène de la molécule B7 co-stimulatrice de cellules T.

10. Vaccins vivants selon la revendication 1 à 3, se caractérisant par le fait que les gènes suicides sont des substances qui transforment les principes actifs en un produit toxique.

11. Vaccins vivants selon la revendication 1 à 3 ou 10, se caractérisant par le fait que le gène suicide est le gène de la thymidine kinase du virus Herpes simplex (HSV-TK) ou le gène de cytosine désaminase.

12. Procédé de fabrication d'un vaccin vivant selon l'une des revendications 1 à 11 dans des systèmes autologues ou allogènes.

13. Vaccins vivants selon l'une des revendications 1 à 11 pour un emploi en tant que médicament.

14. Procédé de fabrication de vaccins vivants selon l'une des revendications 1 à 11 pour le traitement de maladies tumorales.
